# EUROPÄISCHE PATENTSCHRIFT

(11) **EP 0 572 768 B1**
(45) Veröffentlichungstag und Bekanntmachung des Hinweises auf die Patenterteilung: **16.07.1997**
(21) Anmeldenummer: 93104215.4
(22) Anmeldetag: 16.03.1993
(51) Int. Cl.: A61K 7/13

(54) **Verfahren zur Herstellung von pulverförmigen Mitteln zum Färben von menschlichen Haaren**
Process for preparing powdery agents for human hair dyeing
Procédé de préparation d'agents pulvérulents pour la teinture des cheveux humains

(30) Priorität: 30.05.1992 DE 4217918; 12.06.1992 DE 4219181
(43) Veröffentlichungstag der Anmeldung: 08.12.1993
(73) Patentinhaber: GOLDWELL GmbH, D-64297 Darmstadt (DE)
(72) Erfinder: Lorenz, Heribert, W-6101 Gross-Bieberau 1 (DE)

(56) Entgegenhaltungen:
- US-A- 4 183 366

## Beschreibung

Die vorliegende Erfindung betrifft ein neues Verfahren zur Herstellung von pulverförmigen Mitteln zum Färben von menschlichen Haaren mit verbesserten Gebrauchseigenschaften.

Im Zuge der Rückbesinnung auf die Verwendung natürlicher Roh- und Wirkstoffe auch in der Kosmetik wurde seit langem vorgeschlagen, auch Haarfärbemittel auf Basis natürlicher Farbstoffe herzustellen. Als charakteristisches Beispiel für einen bereits seit Jahrtausenden bekannten natürlichen Farbstoff für menschliche Haare sei dabei auf das zur Rotfärbung eingesetzte Henna verwiesen.

Solche bekannten Mittel zum Färben von menschlichen Haaren auf Basis natürlicher Farbstoffe liegen in der Regel als Pulver vor und enthalten, neben den natürlichen Haarfarben und gegebenenfalls auch synthetischen direktziehenden Farbstoffen ein pulverförmiges Trägermaterial. Dieses Pulver wird bei Gebrauch mit Wasser angerührt und auf das Haar aufgebracht.

Die Gebrauchseigenschaften dieser Haarfärbepulver sind jedoch bisher nicht befriedigend. Sie stauben nicht nur bei der Anwendung, sondern sind auch bei der Dosierung nicht exakt handhabbar, wodurch das erwünschte Färbeergebnis beeinträchtigt werden kann.

Es wurde nunmehr gefunden, daß ein pulverförmiges Mittel zum Färben von menschlichen Haaren auf Basis von direktziehenden, insbesondere natürlichen Farbstoffen, das die erwähnten Nachteile nicht aufweist, insbesondere nicht mehr staubt, erhalten werden kann, wenn man das pulverförmige Ausgangsmaterial durch Zusatz eines Binde- bzw. Verdickungsmittels agglomiert, wobei in einer ersten Stufe das pulverförmige Ausgangsmaterial mit einem Bindemittel behandelt und in einer zweiten Stufe ein weiteres Binde- und Verdickungsmittel dem Gemisch zugesetzt und anschließend das erhaltene Agglomerat getrocknet wird.

Auf diese Weise wird ein stabiles staubfreies Produkt erhalten, das sich problemlos vor der Anwendung auf dem Haar mit Wasser homogen vermischen und anschließend auftragen läßt.

Wichtig bei dem zweistufigen Beschichtungs- bzw. Agglomerierungsverfahren nach der Erfindung ist, daß in der zweiten Phase ein Bindemittel eingesetzt wird, das gleichzeitig eine verdickende Wirkung aufweist.

Dabei liegt das Gewichtsverhältnis zwischen dem in der ersten Stufe eingesetzten Bindemittel und dem in der zweiten Stufe zugesetzten Binde- bzw. Verdickungsmittel bei etwa 1 : 2 bis 1 : 50, vorzugsweise 1 : 3 bis 1 : 10, insbesondere 1 : 4 bis 1 : 6.
Die Teilchengröße des erfindungsgemäß hergestellten Färbemittel-Agglomerats liegt dabei zwischen 50 und 1000 µm, vorzugsweise 200 bis 500 µm, die sich, wie bereits ausgeführt, ausgezeichnet eignen, um mit wäßrigen Lösungen zubereitet zu werden.

Geeignete Bindemittel zur Durchführung des erfindungsgemäßen Verfahrens sind im Prinzip alle wasserlöslichen, für diesen Zweck bekannten natürlichen und synthetischen Polymeren.

Als solche sind insbesondere die verschiedenen Cellulose-Derivate, beispielsweise Alkylcellulosen wie Methylcellulose und Ethylcellulose, Hydroxyalkylcellulosen wie Hydroxyethylcellulose, Hydroxypropylcellulose und Methylhydroxypropylcellulose sowie auch Carboxymethylcellulose genannt; weiterhin Stärke und Stärkederivate; Gelatine, natürliche Gummen wie Guar-Gum oder Xanthan-Gum oder auch Alkalialginate und Alginsäureester, beispielsweise Propylenglykolalginat, sowie Polyethylenglykole mit einem Molgewicht ab 10 000.

Geeignete synthetische Polymere sind beispielsweise Polyvinylpyrrolidon, Polyacrylamid, Alkalisalze der Polyacrylsäure, etc. Besonders geeignet als Bindemittel in der ersten Stufe sind Dextrine und Dextrinderivate wie weißes und gelbes Dextrin und Cyclodextrin sowie insbesondere Maltodextrin.

Die obengenannten Bindemittel weisen gleichzeitig eine verdickende Wirkung auf und sind daher geeignet, in beiden Stufen des erfindungsgemäßen Verfahrens zum Einsatz zu gelangen.

Ein besonderes Binde- und Verdickungsmittel, das in der zweiten Verfahrensstufe, vorzugsweise in wäßriger Lösung, zugesetzt wird, besteht vorzugsweise aus wasserlöslichen Pflanzengummen wie Xanthan-Gum und/oder Guar-Gum.

Geeignete Bindemittel, die in der ersten Verfahrensphase zugesetzt werden, jedoch keine oder nur eine geringe verdickende Wirkung aufweisen und deshalb zum Zusatz in der zweiten Verfahrensphase nicht oder nur weniger geeignet sind, sind beispielsweise Acrylestercopolymerisate mit Acrylsäure und/oder Dialkylaminoalkyl(meth)acrylaten, beispielsweise Diethyl- und Dimethylaminoethylmethacrylat, die gegebenenfalls auch quaternisiert sein können, neutralisierte Copolymerisate aus Methacrylsäure und Methylmethacrylat sowie weitere bekannte wasserlösliche Copolymere.

Die Gesamtmenge des aufgebrachten Bindemittels in beiden Herstellungsstufen liegt bei etwa 0,5 bis etwa 20 Gew.-%, insbesondere 2 bis 15 Gew.-%, vorzugsweise 5 bis etwa 10 Gew.-%, der Gesamtzusammensetzung des agglomerierten Farbpulvers.

Verbessert werden können die Gebrauchseigenschaften des erfindungsgemäß hergestellten Farbpulvers noch durch die Mitverwendung geringer Mengen von Tensiden, insbesondere anionischen und nichtionischen Tensiden.

Als solche sind insbesondere die bekannten langkettigen Alkyl- und Alkylethersulfate, Fettsäuretauride und Olefinsulfonate sowie C₁₂-C₁₈-Fettalkohol-Polyglykolether, beispielsweise mit 3 bis 15 Ethylenoxid-Einheiten pro Mol, und Alkylphenolpolyglykolether, beispielsweise Nonylphenolpolyglykolether mit etwa 4 bis etwa 10 Ethylenoxid-Einheiten pro Mol, geeignet.

Deren Anteil liegt vorzugsweise bei etwa 0,1 bis etwa 5 Gew.-%, vorzugsweise 0,15 bis 2,5 Gew.-%, insbesondere bis etwa 1 Gew.-%, berechnet auf die Gesamtzusammensetzung des erfindungsgemäß hergestellten Färbepulvers.

Wie bereits angedeutet, enthalten die Färbemittel die in solchen Mitteln bekannten und üblichen Bestandteile.

Es können die bekannten Naturfarbstoffe eingesetzt werden, beispielsweise Henna (rot, schwarz oder neutral), Alkannin bzw. Alkannawurzelpulver, Blauholzpulver, Krappwurzel- und Rhabarberwurzelpulver, etc.

Zur Nuancierung der mit den Naturfarbstoffen erzielten Farbtöne können erforderlichenfalls auch synthetische direktziehende Farbstoffe, beispielsweise die bekannten "Arianor"-Farbstoffe, mitverwendet werden.
Entsprechende Zusammensetzungen sind Stand der Technik und in einschlägigen Veröffentlichungen beschrieben.

Die Herstellung nach der Erfindung erfolgt im Prinzip auf folgende Weise:
Zunächst wird das pulverförmige Mittel, vorzugsweise in einer Wirbelschichtsprühanlage, mit einer Lösung des Bindemittels, die vorzugsweise alkalisch eingestellt ist, besprüht. Hierzu wird zweckmäßigerweise eine Wirbelschicht-Topsprayanlage mit Unterdruckbetrieb verwendet.

Das Spruhen erfolgt vorzugsweise bei einer Temperatur zwischen etwa 25 und etwa 50 °C, vorzugsweise 30 bis etwa 40 °C.

Nach dem vollständigen Aufbringen der Bindemittel-Lösung wird anschließend, vor dem Trocknen, der Rest des Bindemittels, der gleichzeitig als Verdickungsmittel wirkt, angesaugt und nach kurzem Verwirbeln das erhaltene Produkt getrocknet.

Geeignete Wirbelschichtverfahren sind aus der Literatur hinreichend bekannt, beispielsweise beschreiben Kala, Dittken und Moldenhauer in Pharmazie Nr. 11/1971 ein solches Verfahren.

Ein entsprechendes Verfahren ist auch als "Wurster"-Verfahren bekannt und im Journal of the American Pharmaceutical Association, Vol. 48 (1959), S.451 von D.Wurster beschrieben.

Eine weitere Beschreibung von A. M. Mehta, M.J.Valazza und St.E.Abele findet sich in Pharmaceutical Technology, April 1986, "Evaluation of fluid-bed processes for enteric coating systems".

Im folgenden wird beispielhaft das erfindungsgemäße Verfahren zur Herstellung eines staubfreien Färbepulvers erläutert:

### Beispiel 1

Eine Mischung aus
- 65,0 (Gew.-Teilen): Alkannawurzel
- 8,0: Hennaschwarz
- 2,0: Natriumalginat
- 2,0: Natriumcarboxymethylcellulose
- 2,5: Natriumcarbonat
- 0,5: Natriumlaurylsulfat
wird in eine Wirbelschicht-Topsprayanlage eingebracht und mit 30,8 Gewichtsteilen einer Lösung aus
- 0,42 (Gew.-Teilen): NaOH
- 6,31: Methacrylsäure/Methylmethacrylat-Copolymerisat (Eudragit^{R} L30D)-Dispersion (30 % Feststoff-Gehalt)
in 23,35 Gew.-Teilen Wasser
bei 25 bis 35 °C besprüht.

Nach beendetem Sprühvorgang werden nach 35 Minuten 4,16 Gew.-Teile eines Gemisches aus 0,96 Gew.-Teilen Hydroxyethylcellulose und 3,20 Gew.-Teilen Natriumcarboxymethylcellulose innerhalb 5 Minuten eingezogen und anschließend das erhaltene Agglomerat sofort bei etwa 35° - 45 °C getrocknet.

Das erhaltene Produkt ist absolut staubfrei, besitzt ausgezeichnete Rieselfähigkeit, klebt nicht und läßt sich mit Wasserstoffperoxid-Lösung im Verhältnis 1 : 4 zu einem gut auf das Haar aufzutragenden Färbemittel anrühren, wobei nach 30- bis 60-minütiger Einwirkzeit ein graphitgrauer Farbton erhalten wird.

Jeweils weniger als 1 % der Teilchen weisen einen Durchmesser unterhalb 50 µm und oberhalb 900 µm auf.

### Beispiel 2

Ein Produkt mit annähernd gleich guten Eigenschaften wird erhalten, wenn in der ersten Agglomerierungsstufe eine Mischung aus 8 Gew.-% Polyethylenglykol 10 000/Polyethylenglykol 20 000 im Verhältnis 1 : 1 in 92 Gew.-% eines 50 : 50-Gemisches aus Isopropylalkohol/Wasser aufgesprüht und anschließend ein Hydroxyethylcellulose/Carboxymethylcellulose-Verdickungsmittel-Gemisch entsprechend dem obenbeschriebenen Beispiel zugesetzt wird.

Das als Ausgangsmaterial verwendete Farbpulver weist folgende Zusammensetzung auf:
- 67,0 (Gew.-Teile): Hennarot
- 8,0: Hennaschwarz
- 2,0: Basic Blue 99
- 1,5: Natriumalginat
- 1,5: Natriumcarboxymethylcellulose.

Das erhaltene Agglomerat läßt sich mit Wasser im Verhältnis 1 : 4 gut anrühren und ergibt nach 30- bis 60-minütiger Einwirkung auf dem Haar einen dunkelbraunen Farbton.

### Beispiel 3

Eine Mischung aus
- 2,70 (Gew.-Teilen): Hennarot
- 0,60: Hennaschwarz
- 8,22: Blauholz
- 0,23: Natriumalginat
- 0,25: Natriumcarboxymethylcellulose
- 0,05: Natriumcarbonat
wird in eine Wirbelschicht-Topsprayanlage eingebracht und mit 3,52 Gewichtsteilen einer Lösung aus
0,32 (Gew.-Teilen) Maltodextrin
in 3,20 Gew.-Teilen Wasser
bei 25 bis 35 °C besprüht.

Nach beendetem Sprühvorgang werden nach etwa 20 Minuten 13,55 Gew.-Teile einer Lösung aus 0,20 Gew.-Teilen Xanthan-Gum in 13,35 Gew.-Teilen Wasser innerhalb etwa 30 Minuten aufgesprüht und anschließend das erhaltene Agglomerat sofort bei etwa 35° - 45 °C getrocknet.

Das erhaltene Produkt ist absolut staubfrei, besitzt ausgezeichnete Rieselfähigkeit, klebt nicht und läßt sich mit Wasser im Verhältnis 1 : 4 zu einem gut auf das Haar aufzutragenden Färbemittel anrühren, wobei nach 30- bis 60-minütiger Einwirkzeit ein rotbrauner Farbton erhalten wird.

Jeweils weniger als 1 % der Teilchen weisen einen Durchmesser unterhalb 50 µm und oberhalb 900 µm auf.

## Patentansprüche

1. Verfahren zur Herstellung von nichtstaubenden pulverförmigen Mitteln zum Färben von menschlichen Haaren, wobei das pulverförmige Ausgangsmaterial durch Zusatz eines Binde- bzw. Verdickungsmittels agglomeriert wird, dadurch gekennzeichnet, daß das mindestens einen direktziehenden Farbstoff, insbesondere einen Naturfarbstoff enthaltende pulverförmige Ausgangsmaterial in einer ersten Stufe mit einem Bindemittel behandelt und in einer zweiten Stufe ein weiteres Binde- und Verdickungsmittel dem Gemisch zugesetzt und anschließend das erhaltene Agglomerat getrocknet wird.

2. Verfahren nach Anspruch 1, dadurch gekennzeichnet, daß das Gewichtsverhältnis des in der ersten Stufe eingesetzten Bindemittels zu dem in der zweiten Stufe zugesetzten Binde- und Verdickungsmittel bei 1 : 2 bis 1 : 50 liegt.

3. Verfahren nach Anspruch 2, dadurch gekennzeichnet, daß das Verhältnis des in der ersten Stufe eingesetzten Bindemittels zu dem in der zweiten Stufe zugesetzten Binde- und Verdickungsmittel bei 1 : 3 bis 1 : 10 liegt.

4. Verfahren nach Anspruch 3, dadurch gekennzeichnet, daß das Gewichtsverhältnis des in der ersten Stufe eingesetzten Bindemittels zu dem in der zweiten Stufe zugesetzten Binde- und Verdickungsmittel bei etwa 1 : 4 bis 1 : 6 liegt.

5. Verfahren nach einem der vorhergehenden Ansprüche, dadurch gekennzeichnet, daß das in der ersten Stufe eingesetzte Bindemittel und das in der zweiten Stufe zugesetzte Binde- und Verdickungsmittel identisch sind.

6. Verfahren nach einem der Ansprüche 1 bis 4, dadurch gekennzeichnet, daß es sich bei dem in der ersten Stufe eingesetzten Bindemittel und dem in der zweiten Stufe zugesetzten Binde- und Verdickungsmittel um verschiedene Substanzen handelt.

7. Verfahren nach Anspruch 6, dadurch gekennzeichnet, daß es sich bei dem in der ersten Stufe eingesetzten Bindemittel um ein neutralisiertes Copolymerisat aus Methacrylsäure und Methylmethacrylat handelt.

8. Verfahren nach einem der vorhergehenden Ansprüche, dadurch gekennzeichnet, daß es sich bei dem in der zweiten Stufe zugesetzten Binde- und Verdickungsmittel um ein oder mehrere wasserlösliche Cellulosederivate handelt.

9. Verfahren nach einem oder mehreren der Ansprüche 1 bis 6, dadurch gekennzeichnet, daß das in der ersten Stufe aufgebrachte Bindemittel aus Dextrin und/oder einem Dextrinderivat besteht.

10. Verfahren nach Anspruch 9, dadurch gekennzeichnet, daß das Bindemittel Maltodextrin enthält.

11. Verfahren nach Anspruch 9 oder 10, dadurch gekennzeichnet, daß das in der zweiten Stufe eingesetzte Bindemittel aus wasserlöslichen Gummen besteht.

12. Verfahren nach Anspruch 11, dadurch gekennzeichnet, daß das in der zweiten Stufe eingesetzte Bindemittel aus Xanthan-Gum besteht.

13. Verfahren nach Anspruch 11, dadurch gekennzeichnet, daß das in der zweiten Stufe eingesetzte Bindemittel aus Guar-Gum besteht.

## Claims

1. Process for the production of non-dusting pulverulent compositions for direct dyeing of human hair wherein the powdery basic substance is agglomerated by the addition of a binding or thickening agent, characterized in that the powdery basic substance containing at least one direct dyestuff is treated with a binding agent in a first stage, and in a second stage another binding and thickening agent is added to the mixture, whereupon the agglomerate thus obtained is dried.

2. Process according to claim 1, characterized in that the weight proportion of the binding agent used in the first stage to the binding and thickening agent added in the second stage is 1 : 2 to 1 : 50.

3. Process according to claim 2, characterized in that the proportion of the binding agent used in the first stage to the binding and thickening agent used in the second stage is 1 : 3 to 1 : 10.

4. Process according to claim 3, characterized in that the weight proportion of the binding agent used in the first stage to the binding and thickening agent used in the second stage is about 1 : 4 to 1 : 6.

5. Process according to one of the preceding claims, characterized in that the binding agent used in the first stage and the binding and thickening agent added in the second stage are identical.

6. Process according to one of claims 1 to 4, characterized in that the binding agent used in the first stage and the binding and thickening agent added in the second stage are different.

7. Process according to claim 6, characterized in that the binding agent used in the first stage is a neutralized copolymer of methacrylic acid and methyl methacrylate.

8. Process according to one of the preceding claims, characterized in that the binding and thickening agent added in the second stage comprises one or several water-soluble cellulose derivatives.

9. Process according to one or more of claims 1 to 6, characterized in that the binding agent used in the first stage comprises dextrin and (or) a derivative thereof.

10. Process according to claim 9, chararacterized in that the binding agent comprises maltodextrin.

11. Process according to one or more of claims 1 to 10, characterized in that the binding agent added in the second stage is a water-soluble gum.

12. Process according to claim 11, characterized in that the binding agent added in the second stage is xanthan gum.

13. Process according to claim 11, characterized in that the binding agent used in the second stage is guar gum.

## Revendications

1. Procédé pour la fabrication de produits pulvérulents ne formant pas de poussière, destinés à la teinture des cheveux humains, dans lequel le produit pulvérulent de départ est aggloméré par addition d'un liant ou épaississant, caractérisé en ce que, dans une première étape, on traite par un liant le produit de départ pulvérulent, contenant au moins un colorant direct, en particulier un colorant naturel, et, dans une seconde étape, on ajoute au mélange un autre liant et épaississant, l'agglomérat obtenu étant ensuite séché.

2. Procédé selon la revendication 1, caractérisé en ce que le rapport pondéral du liant utilisé dans la première étape au liant et épaississant ajouté dans la seconde étape va de 1:2 à 1:50.

3. Procédé selon la revendication 2, caractérisé en ce que le rapport du liant utilisé dans la première étape au liant et épaississant ajouté dans la seconde étape va de 1:3 à 1:10.

4. Procédé Selon la revendication 3, caractérisé en ce que le rapport pondéral du liant utilisé dans la première étape au liant et épaississant ajouté dans la seconde étape va d'environ 1:4 à 1:6.

5. Procédé selon l'une des revendications précédentes, caractérisé en ce que le liant utilisé dans la première étape et le liant et épaississant ajouté dans la seconde étape sont identiques.

6. Procédé selon l'une des revendications 1 à 4, caractérisé par le fait que le liant utilisé dans la première étape et le liant et épaississant ajouté dans la seconde étape, sont des substances différentes.

7. Procédé selon la revendication 6, caractérisé par le fait que le liant utilisé dans la première étape, est un copolymère neutralisé d'acide méthacrylique et de méthacrylate de méthyle.

8. Procédé selon l'une des revendications précédentes, caractérisé par le fait que le liant et épaississant ajouté dans la seconde étape est constitué d'un ou de plusieurs dérivés de cellulose, solubles dans l'eau.

9. Procédé selon une ou plusieurs des revendications 1 à 6, caractérisé en ce que le liant introduit dans la première étape est constitué de dextrine et/ou d'un dérivé de dextrine.

10. Procédé selon la revendication 9, caractérisé en ce que le liant contient de la maltodextrine.

11. Procédé selon la revendication 9 ou 10, caractérisé en ce que le liant utilisé dans la seconde étape est constitué de gommes solubles dans l'eau.

12. Procédé selon la revendication 11, caractérisé en ce que le liant utilisé dans la seconde étape est constitué de gomme de xanthane.

13. Procédé selon la revendication 11, caractérisé en ce que le liant utilisé dans la seconde étape est constitué de gomme guar.
